# EUROPEAN PATENT APPLICATION

(11) **EP 3 267 219 A1**
(43) Date of publication of application: **10.01.2018**
(21) Application number: 17150111.7
(22) Date of filing: 03.01.2017
(51) Int. Cl.: G01S 7/52, G01S 15/89, G10K 11/34

(54) **ULTRASOUND PROBE, ULTRASOUND DIAGNOSTIC APPARATUS, AND METHOD OF CONTROLLING ULTRASOUND DIAGNOSTIC APPARATUS**

(30) Priority: 07.07.2016 KR 20160086262
(71) Applicant: Samsung Medison Co., Ltd., Hongcheon-gun, Gangwon-do, 25108 (KR); Sogang University Research Foundation, Seoul 04107 (KR)
(72) Inventor: KIM, Kang-Sik, Gyeonggi-do (KR); SONG, Tae-kyong, Seoul (KR); KANG, Hyun-gil, Seoul (KR); PARK, Ji-Won, Seoul (KR)
(74) Representative: D'Halleweyn, Nele Veerle Trees Gertrudis

(57) **Abstract**

An ultrasound probe includes a plurality of transducer elements (311) configured to transmit ultrasound waves to an object and receive ultrasound echo signals corresponding to the transmitted ultrasound waves from the object, wherein the plurality of transducer elements (311) are classified to be included in a plurality of first sub-arrays (310a, 310b, 310c), a plurality of first analog beamformers (330a, 330b, 330c) configured to generate first synthesized signals by performing first beamforming on each of the ultrasound echo signals received by the plurality of transducer elements (311) included in each of the plurality of the first sub-arrays (310a, 310b, 310c), and a second analog beamformer (340) configured to generate a second synthesized signal by performing second beamforming on the first synthesized signals generated by the plurality of first analog beamformers (330a, 330b, 330c).

## Description

### CROSS-REFERENCE TO RELATED APPLICATION

This application claims priority from Korean Patent Application No. 10-2016-0086262, filed on July 7, 2016, in the Korean Intellectual Property Office, the disclosure of which is incorporated herein in its entirety by reference.

### BACKGROUND

### 1. Field

One or more embodiments relate to an ultrasound probe, an ultrasound diagnosis apparatus, and a method of controlling an ultrasound diagnosis apparatus.

### 2. Description of the Related Art

Ultrasound diagnostic apparatuses transmit ultrasound signals generated by transducers of a probe to an object and detect information about signals reflected from the object, thereby obtaining at least one image of an internal part, for example, soft tissue or blood flow, of the object.

An ultrasound diagnosis apparatus performs a beamforming process on the signals reflected from the object. The beamforming process is a signal processing technology of synthesizing multichannel electric signals to generate a single signal.

The ultrasound diagnosis apparatus may convert ultrasound waves detected by each of a plurality of transducer elements to electric signals, and adjust and synthesize a delay between the multichannel electric signals, thereby performing the beamforming process.

Digital beamformers, which perform beamforming in a digital method, convert analog echo signals detected by each of the transducer elements to digital signals and perform beamforming and signal processing. Accordingly, an analog-to-digital converter is essentially used for each transducer element. Also, when an ultrasound echo signal is detected through a two-dimensional transducer array, the same number of analog-to-digital converters as the number of transducer elements used for detecting the ultrasound echo signals is needed. Thus, the size of hardware used for the digital beamforming is very large and the complexity of hardware increases.

Analog beamformers perform beamforming by applying different delays to analog echo signals detected by the transducer elements and synthesizing delayed analog echo signals on the same time altogether in an analog method, and convert analog signals, on which the beamforming is performed, to digital signals. The analog beamformer requires analog RAM that applies different delays to ultrasound echo signals detected by the transducer elements. In response to an applied system signal, a plurality of capacitors included in the sample/hold circuit sequentially perform sampling and reading out. As the capacitors sequentially perform sampling and reading out, the ultrasound echo signals detected by the transducer elements are delayed.

When the number of capacitors of the analog RAM included in the analog beamformer is less than that of capacitors of analog RAM that are needed to perform accurate analog beamforming, analog beamforming is not performed appropriately. If so, the resolution of an image generated by the ultrasound diagnosis apparatus is lowered. However, since the analog beamformer cannot include unlimitedly analog RAM having a plurality of capacitors due to a spatial restriction, the resolution of an image generated by the ultrasound diagnosis apparatus may be lowered.

### SUMMARY

One or more embodiments include an ultrasound probe including an analog beamformer, which may address a spatial restriction by reducing the number of capacitors of analog RAM needed to perform analog beamforming.

One or more embodiments include an ultrasound diagnosis apparatus, which may generate an image of which the resolution is not lowered even when the number of capacitors of analog RAM is reduced, and a method of controlling the ultrasound diagnosis apparatus.

Additional aspects will be set forth in part in the description which follows and, in part, will be apparent from the description, or may be learned by practice of the presented embodiments.

According to one or more embodiments, an ultrasound probe includes a plurality of transducer elements configured to transmit ultrasound waves to an object and receive ultrasound echo signals corresponding to the transmitted ultrasound waves from the object, wherein the plurality of transducer elements are classified to be included in a plurality of first sub-arrays, a plurality of first analog beamformers configured to generate first synthesized signals by performing first beamforming on each of the ultrasound echo signals received by the plurality of transducer elements included in each of the plurality of the first sub-arrays, and a second analog beamformer configured to generate a second synthesized signal by performing second beamforming on the first synthesized signals generated by the plurality of first analog beamformers.

The second analog beamformer may be provided in a plural number. The first synthesized signals generated by the plurality of first analog beamformers may be classified to be input to one of the plurality of second analog beamformers. Each of the plurality of second analog beamformers may generate a second synthesized signal by performing second beamforming on the first synthesized signals. The ultrasound probe may further include a third analog beamformer configured to generate a third synthesized signal by performing third beamforming on the second synthesized signals generated by the plurality of second analog beamformers.

Each of the plurality of first analog beamformers may include a first analog RAM including a plurality of capacitors to secure a difference in a signal delay time between the plurality of transducer elements in each of the plurality of first sub-arrays while first beamforming is performed. The number of the plurality of transducer elements included in each of the first sub-arrays may be determined based on the number of the plurality of capacitors included in the first analog RAM.

The second analog beamformer may include a second analog RAM including a plurality of capacitors to secure a difference in a signal delay time between the plurality of first sub-arrays while second beamforming is performed. The number of the first sub-arrays may be determined based on the number of the plurality of capacitors included in the second analog RAM.

The number of the plurality of transducer elements included in each of the plurality of first sub-arrays may be determined based on at least one of a total number of the plurality of transducer elements, a distance between the plurality of transducer elements, and a read-out sampling rate of the plurality of transducer elements.

The number of the plurality of first sub-arrays may be determined based on at least one of the number of the plurality of transducer elements, a distance between the plurality of transducer elements, and a read-out sampling rate of the plurality of transducer elements.

The plurality of transducer elements may be classified to be included in the plurality of first sub-arrays based on a distance between a focal point on which the ultrasound waves transmitted to the object are focused and each of the plurality of transducer elements.

The plurality of first sub-arrays may be determined based on a first difference value that is a difference between a maximum value and a minimum value of a distance between the focal point and each of the plurality of transducer elements included in each of the first sub-arrays.

Each of the plurality of first sub-arrays may be determined such that the first difference value is within an error range.

The plurality of first analog beamformers may include a first analog RAM including a plurality of capacitors to secure a difference in a signal delay time between the plurality of transducer elements in each of the plurality of first sub-arrays while first beamforming is performed. The second analog beamformer may include a second analog RAM including a plurality of capacitors to secure a difference in a signal delay time between the plurality of first sub-arrays while second beamforming is performed. The number of the plurality of transducer elements included in one of the plurality of first sub-arrays may be determined based on a sum of the number of the plurality of capacitors included in the first analog RAM and the number of the plurality of capacitors included in the second analog RAM.

The plurality of first analog beamformers may include a first analog RAM including a plurality of capacitors to secure a difference in a signal delay time between the plurality of transducer elements in each of the plurality of first sub-arrays while first beamforming is performed. The second analog beamformer may include a second analog RAM including a plurality of capacitors to secure a difference in a signal delay time between the plurality of first sub-arrays while second beamforming is performed. The number of the plurality of first sub-arrays may be determined based on a sum of the number of the plurality of capacitors included in the first analog RAM and the number of the plurality of capacitors included in the second analog RAM.

According to one or more embodiments, an ultrasound diagnosis apparatus includes a plurality of transducer elements configured to transmit ultrasound waves to an object and receive ultrasound echo signals corresponding to the transmitted ultrasound waves from the object, wherein the plurality of transducer elements are classified to be included in a plurality of first sub-arrays, a plurality of first analog beamformers configured to generate first synthesized signals by performing first beamforming on each of the ultrasound echo signals detected by the plurality of transducer elements included in each of the plurality of the first sub-arrays, a second analog beamformer configured to generate a second synthesized signal by performing second beamforming on the first synthesized signals generated by the plurality of first analog beamformers, and a controller configured to determine the transducer elements used to detect the ultrasound echo signals based on a position of a focal point on which the ultrasound waves transmitted to the object are focused.

The plurality of transducer elements may be classified to be included in the plurality of first sub-arrays based on a distance between the focal point on which the ultrasound waves transmitted to the object are focused and each of the plurality of transducer elements determined to be used to detect the ultrasound echo signals.

The number of the plurality of first sub-arrays and the number of the plurality of transducer elements included in one of the plurality of first sub-arrays may be determined based on the number of the plurality of transducer elements determined to be used to detect the ultrasound echo signals.

The number of the plurality of transducer elements to detect the ultrasound echo signals may decrease as the focal point approaches a transducer array including the plurality of transducer elements.

According to one or more embodiments, a method of controlling an ultrasound diagnosis apparatus, which includes transmitting ultrasound waves to an object, detecting ultrasound echo signals corresponding to the transmitted ultrasound waves from the object, determining a plurality of transducer elements used to detect the ultrasound echo signals corresponding to the ultrasound waves reflected from the object based on a position of a focal point on which the ultrasound waves transmitted to the object are focused, classifying the plurality of transducer elements determined to be included in a plurality of first sub-arrays based on the position of the focal point, generating a plurality of first synthesized signals by performing first beamforming on each of the ultrasound echo signals detected by the plurality of transducer elements included in each of the plurality of first sub-arrays, and generating a second synthesized signal by performing second beamforming on the first synthesized signals generated by the plurality of first analog beamformers.

The plurality of transducer elements may be classified to be included in the plurality of first sub-arrays based on a distance between the focal point on which the ultrasound waves transmitted to the object are focused and each of the plurality of transducer elements determined to be used to detect the ultrasound echo signals.

The number of the plurality of first sub-arrays and the number of the plurality of transducer elements included in one of the plurality of first sub-arrays may be determined based on the number of the plurality of transducer elements determined to be used to detect the ultrasound echo signals.

The number of the plurality of transducer elements to detect the ultrasound echo signals may decrease as the focal point approaches a transducer array including the plurality of transducer elements.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating an ultrasound diagnosis apparatus according to an exemplary embodiment;
FIGS. 2A, 2B, and 2C are diagrams respectively illustrating an ultrasound diagnosis apparatus according to an exemplary embodiment;
FIG. 3 is a block diagram illustrating a configuration of a probe of an ultrasound diagnosis apparatus according to an embodiment;
FIG. 4 is a block diagram illustrating in detail a configuration of a probe of an ultrasound diagnosis apparatus according to an embodiment;
FIG. 5 is a circuit diagram of analog RAM included in an analog beamformer according to an embodiment;
FIG. 6 is a conceptual diagram of a relationship between the number of capacitors included in the analog RAM and delay times of ultrasound echo signals detected by each of a plurality of transducer elements;
FIG. 7 is a block diagram illustrating a configuration of a probe of the ultrasound diagnosis apparatus including an analog beamformer of a triple structure, according to an embodiment;
FIG. 8 is a graph of the number of capacitors needed for analog beam forming to an analog beamformer having a multi-structure and the number of transducer elements;
FIG. 9 is a graph of the number of capacitors needed for analog beamforming to the number of transducer elements included in a first sub-array, in a probe of an ultrasound diagnosis apparatus including an analog beamformer having a dual structure according to an embodiment;
FIG. 10 is a conceptual diagram illustrating classification of the first sub-arrays with respect to a distance between a focal point and each of a plurality of transducer elements, in a probe of an ultrasound diagnosis apparatus including an analog beamformer having a dual structure according to an embodiment;
FIG. 11 is a conceptual diagram illustrating classification of the first sub-arrays with respect to a distance between a focal point and each of a plurality of transducer elements, in a probe of an ultrasound diagnosis apparatus including an analog beamformer having a dual structure according to an embodiment;
FIG. 12 is a conceptual diagram illustrating determination of a transducer element for detecting ultrasound echo signals based on a distance between the focal point and a transducer array, in a probe of a diagnosis apparatus according to an embodiment;
FIG. 13 is a conceptual diagram illustrating determination of the transducer element for detecting ultrasound echo signals based on a distance between the focal point and a transducer array, in a probe of a diagnosis apparatus according to an embodiment;
FIG. 14 is a conceptual diagram illustrating determination of the first sub-array and the transducer element for detecting ultrasound echo signals based on a distance between the focal point and the transducer array, in a probe of a diagnosis apparatus including an analog beamformer having a dual structure according to an embodiment;
FIG. 15 is a conceptual diagram illustrating determination of the first sub-array and the transducer element for detecting ultrasound echo signals based on a distance between the focal point and the transducer array, in a probe of a diagnosis apparatus including an analog beamformer having a dual structure according to an embodiment;
FIG. 16 is a block diagram of an ultrasound diagnosis apparatus including an analog beamformer having a dual structure according to an embodiment; and
FIG. 17 is a flowchart of a method of controlling an ultrasound diagnosis apparatus including and analog beamformer having a dual structure, according to an embodiment.

### DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

Terms such as "part" and "portion" used herein denote those that may be embodied by software or hardware. According to exemplary embodiments, a plurality of parts or portions may be embodied by a single unit or element, or a single part or portion may include a plurality of elements.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

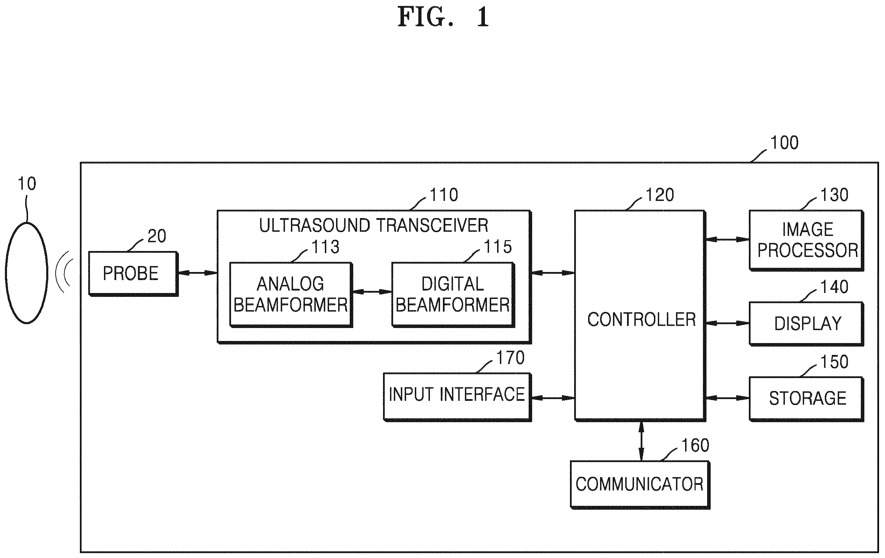

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

In the present embodiment, the probe 20 may include a plurality of transducers. The transducers are arranged in two dimensions (2D), forming a 2D transducer array.

For example, the 2D transducer array may include a plurality of sub-arrays arranged in a first direction, each of the sub-arrays including a plurality of transducers arranged in a second direction that is different from the first direction.

The ultrasound transceiver 110 may include an analog beamformer 113 and a digital beamformer 115. Although FIG. 1 illustrates that the ultrasound transceiver 110 and the probe 20 are provided as being separate from each other, the probe 20 according to the present exemplary embodiment may include the entire ultrasound transceiver 110 or a part of the ultrasound transceiver 110. For example, the probe 20 may include one or both of the analog beamformer 113 and the digital beamformer 115.

The controller 120 may calculate a time delay value for digital beamforming with respect to the sub-arrays included in the 2D transducer array. Also, the controller 120 may calculate a time delay value for analog beamforming for each of the transducers included in any one sub-array of the sub-arrays.

The controller 120 may control the analog beamformer 113 and the digital beamformer 115 to form a transmission signal to be applied to each of the transducers, according to the time delay values for analog beamforming and digital beamforming.

Also, the controller 120 may control the analog beamformer 113 to add signals received from the transducers for each sub-array, according to the time delay value for analog beamforming. Also, the controller 120 may control the ultrasound transceiver 110 to perform analog to digital conversion of the signals added for each sub-array. Also, the controller 120 may control the digital beamformer 115 to generate ultrasound data by adding the digitized signals according to the time delay value for digital beamforming.

Also, the controller 120 may control the analog beamformer 113 to classify the transducers to be included in the sub-arrays, apply the time delay value for performing analog beamforming, and add the signals for each of the sub-arrays. Also, the controller 120 may control the analog beamformer 113 to add again synthesized signals generated by adding the signals for each sub-array by applying the time delay value for performing analog beamforming.

The image processor 130 generates an ultrasound image by using generated ultrasound data.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160.

A program for controlling the ultrasound diagnosis apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

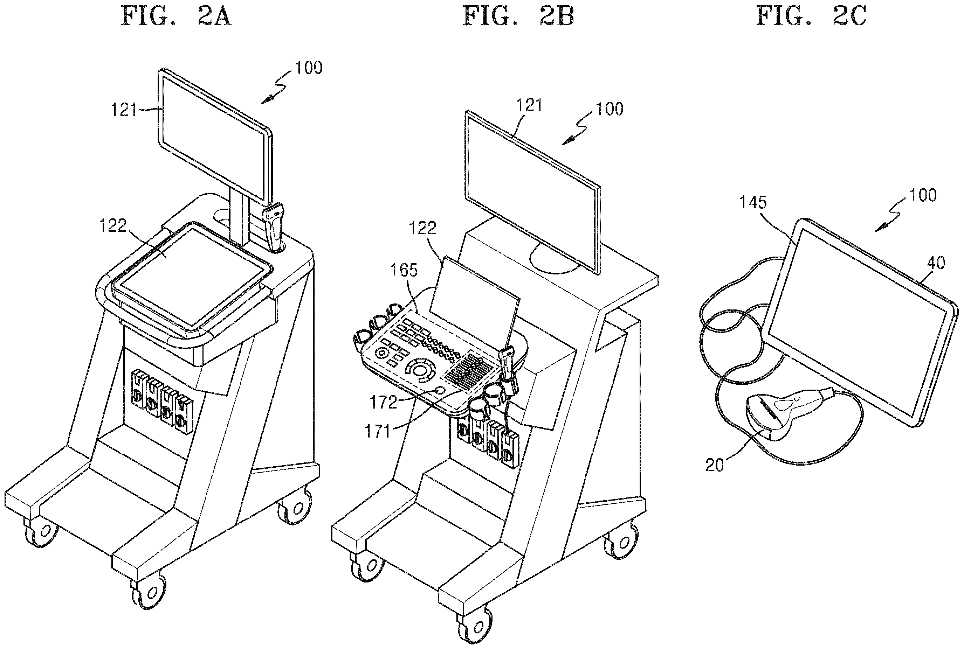

An example of the ultrasound diagnosis apparatus 100 according to the present exemplary embodiment is described below with reference to FIGS. 2A, 2B, and 2C.

FIGS. 2A, 2B, and 2C are diagrams illustrating ultrasound diagnosis apparatus according to an exemplary embodiment.

Referring to FIGS. 2A and 2B, the ultrasound diagnosis apparatus 100 may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100. The main display 121 and the sub-display 122 may provide graphical user interfaces (GUI), thereby receiving user's inputs of data to control the ultrasound diagnosis apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100 may control the display of the ultrasound image on the main display 121 by using the input control data.

Referring to FIG. 2B, the ultrasound diagnosis apparatus 100 may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100 may keep displaying a frame image at that time point.

The buttons, trackballs, jog switches, and knobs included in the control panel 165 may be provided as a GUI to the main display 121 or the sub-display 122.

Referring to FIG. 2C, the ultrasound diagnosis apparatus 100 may include a portable device. An example of the portable ultrasound diagnosis apparatus 100 may include, for example, smart phones including probes and applications, laptop computers, personal digital assistants (PDAs), or tablet PCs, but an exemplary embodiment is not limited thereto.

The ultrasound diagnosis apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100, and a GUI.

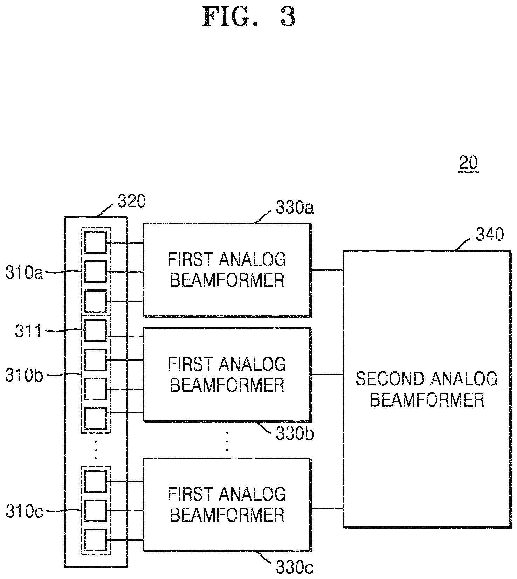

FIG. 3 is a block diagram illustrating a configuration of the probe 20 of the ultrasound diagnosis apparatus 100 according to an embodiment.

Referring to FIG. 3, the ultrasound diagnosis apparatus 100 may include the probe 20 that transmits ultrasound waves to an object 10 (see FIG. 1) and detects ultrasound echo signals corresponding to the ultrasound waves reflected from the object 10.

The probe 20 may include a plurality of transducer elements 311 that transmit ultrasound waves to the object 10 and detect ultrasound echo signals corresponding to the ultrasound waves reflected from the object 10. Also, the probe 20 may include a transducer array 320 that includes the transducer elements 311. In the transducer array 320, the transducer elements 311 may be arranged in one dimension (1D) or in 2D.

The probe 20 according to the present embodiment may include a plurality of analog beamformers, for example, first analog beamformers 330a, 330b, and 330c and a second analog beamformer 340, which perform analog beamforming on the ultrasound echo signals detected by the transducer elements 311, according to the form of embodiment.

The analog beamformers 330 and 340 may have a multi-structure. For example, the ultrasound echo signals detected by the transducer elements 311 may be input to first analog beamformers 330a, 330b, and 330c. The first analog beamformers 330a, 330b, and 330c may perform first beamforming on the ultrasound echo signals detected by the transducer elements 311, thereby generating first synthesized signals. The first synthesized signals generated by the first analog beamformers 330a, 330b, and 330c may be input to the analog beamformers 330 and 340. The second analog beamformer 340 may perform second beamforming on the input first synthesized signals, thereby generating a second synthesized signal.

According to another embodiment, the beamformer may have a multi-structure of three or more levels (stages). For example, when a beamformer is embodied having a triple structure, a synthesized signal output from a first analog beamformer is input to a second analog beamformer, a synthesized signal output from the second analog beamformer is input to a third analog beamformer, and then the third analog beamformer may output a beamformed synthesized signal.

Also, although in the present embodiment the probe 20 is described as having three first analog beamformers 330a, 330b, and 330c, the probe 20 may include less than or more than three first analog beamformers.

To perform multi-structured analog beamforming, a plurality of transducer elements may be classified and included in a plurality of first sub-arrays 310a, 310b, and 310c. Although in the present embodiment the probe 20 is described as having the three first sub-arrays 310a, 310b, and 310c, the probe 20 may include less than or more than three first sub-arrays.

According to an embodiment, the number of transducer elements included in each of the first sub-arrays 310a, 310b, and 310c may be different for each first sub-array. Referring to FIG. 3, the first sub-array 310a may include three transducer elements 311, the first sub-array 310b may include four transducer elements 311, and the first sub-array 310c may include three transducer elements 311. Although in the present embodiment each of the first sub-arrays 310a, 310b, and 310c includes three or four transducer elements 311 for convenience of explanation, the present disclosure is not limited thereto.

To perform the multi-structured analog beamforming, the first analog beamformers 330a, 330b, and 330c may correspond to the first sub-arrays 310a, 310b, and 310c, respectively. For example, the first sub-array 310a may correspond to the first analog beamformer 330a, the first sub-array 310b may correspond to the first analog beamformer 330b, and the first sub-array 310c may correspond to the first analog beamformer 330c. Although the three first sub-arrays 310a, 310b, and 310c and the three first analog beamformers 330a, 330b, and 330c included in the probe 20 are described in the present embodiment, the present disclosure is not limited thereto. Also, although the number of first sub-arrays 310a, 310b, and 310c and the number of first analog beamformers 330a, 330b, and 330c in the probe 20 are the same in the present embodiment, the number of first sub-arrays 310a, 310b, and 310c and the number of first analog beamformers 330a, 330b, and 330c may be different from each other.

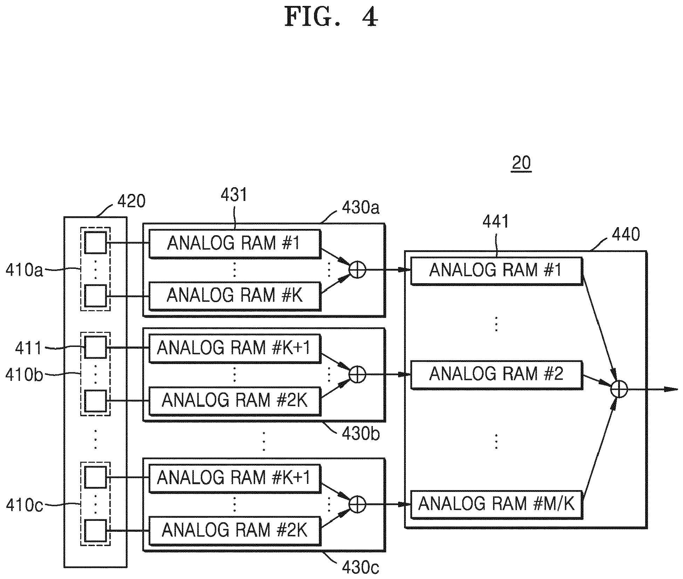

FIG. 4 is a block diagram illustrating in detail a configuration of the probe 20 of the ultrasound diagnosis apparatus 100 according to an embodiment.

Referring to FIG. 4, the probe 20 of the present embodiment may include a plurality of transducer elements 411, a transducer array 420, a plurality of first analog beamformers 430a, 430b, and 430c, and a second analog beamformer 440.

In the transducer array 420, the transducer elements 411 are arranged in 1D or 2D.

The first analog beamformers 430a, 430b, and 430c may correspond to a plurality of first sub-arrays 410a, 410b, and 410c, respectively, in which the transducer elements 411 are classified and included. Also, the first analog beamformers 430a, 430b, and 430c may perform first beamforming on ultrasound echo signals detected by the transducer elements 411 included in the first sub-arrays 410a, 410b, and 410c, thereby generating first synthesized signals.

The second analog beamformer 440 may perform second beamforming on the first synthesized signals generated by the first analog beamformers 430a, 430b, and 430c, thereby generating a second synthesized signal.

Each of the first analog beamformers 430a, 430b, and 430c may include a plurality of first analog RAMs 431 corresponding to each of the transducer elements 411.

The first analog RAMs 431 may be used to secure a delay time between the ultrasound echo signals detected by the transducer elements included in each of the first sub-arrays 410a, 410b, and 410c while each of the first analog beamformers 430a, 430b, and 430c is performing first beamforming.

The second analog beamformer 440 may include a plurality of second analog RAMs 441 respectively corresponding to the first analog beamformers 430a, 430b, and 430c.

The second analog RAMs 441 may be used to secure a signal delay time between the first synthesized signals generated by the first sub-arrays 410a, 410b, and 410c while the second analog beamformer 440 is performing second beamforming.

Referring to FIG. 4, each of the first sub-arrays 410a, 410b, and 410c of the present embodiment may include a K number of transducer elements 411, where K is a natural number. However, each of the first sub-arrays 410a, 410b, and 410c may include less than or more than the K number of transducer elements 411, or a different number of transducer elements 411. Although in the present embodiment the first sub-arrays 410a, 410b, and 410c are indicated to be M-numbered, the first sub-arrays 410a, 410b, and 410c may be present by less than or more than the M-number.

Referring to FIG. 4, the first analog beamformers 430a, 430b, and 430c may correspond to the first sub-arrays 410a, 410b, and 410c, respectively. Each of the first analog beamformers 430a, 430b, and 430c may include the first analog RAMs 431 corresponding to the transducer elements 411 included in each of the first sub-arrays 410a, 410b, and 410c.

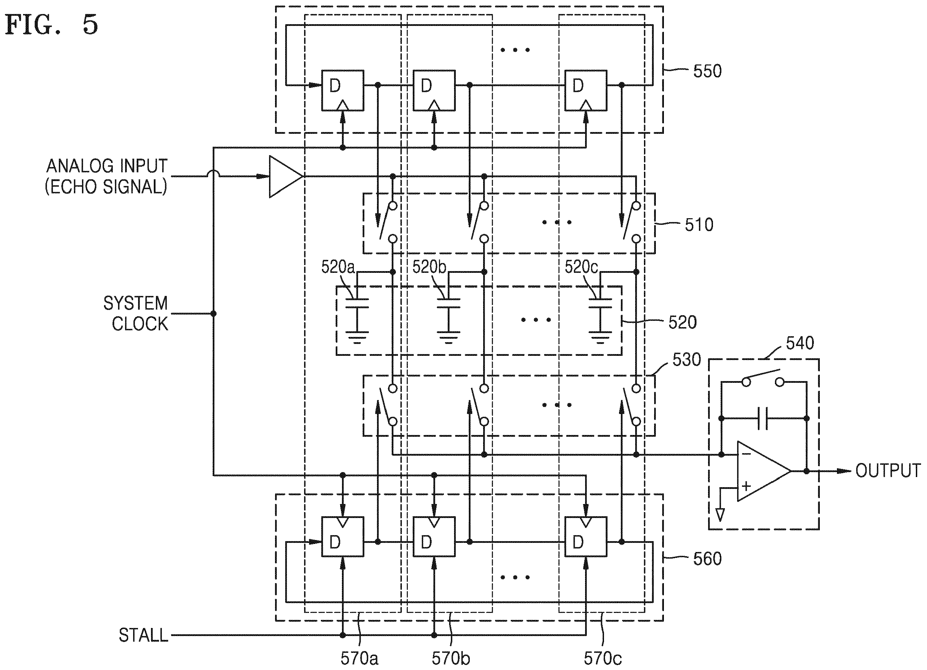

FIG. 5 is a circuit diagram of the first and second analog RAMs 431 and 441 included in an analog beamformer according to an embodiment.

Referring to FIG. 5, the first analog RAM 431 and the second analog RAM 441 may use a sample/hold circuit using capacitors. The sample/hold circuit may include a sample switch 510, a read-out switch 530, a capacitor 520, a charge integrator 540, a first shift register 550, and a second shift register 560. All of the first analog RAMs 431 may be configured by the same circuit. The first analog RAMs 431 and the second analog RAMs 441 may be configured by the same circuit.

The sample switch 510 of the capacitor 520 may be controlled by the first shift register 550. The read-out switch 530 may be controlled by the second shift register 560. The first shift register 550 may be controlled by a system clock signal. The second shift register 560 may be controlled by a stall signal output by the processor.

When a system clock signal is applied to the sample/hold circuit, the position of an output at each rising edge of the system clock signal is sequentially moved from a first operation 570a to a second operation 570b and an n-th operation 570c. As the position of an output is sequentially moved, the sample/hold circuit may sequentially perform sampling and read-out from a first operation capacitor 520a to a second operation capacitor 520b and an n-th operation capacitor 520c.

The first analog beamformers 430a, 430b, and 430c may perform analog beamforming by differing a hold time that is a difference between a sampling time and a read-out time by using the first analog RAMs 431 corresponding to the transducer elements 411.

The second analog beamformer 440 may perform analog beamforming by differing the hold time that is a difference between the sampling time and the read-out time by using the second analog RAMs 441 corresponding to the first analog beamformers 430a, 430b, and 430c.

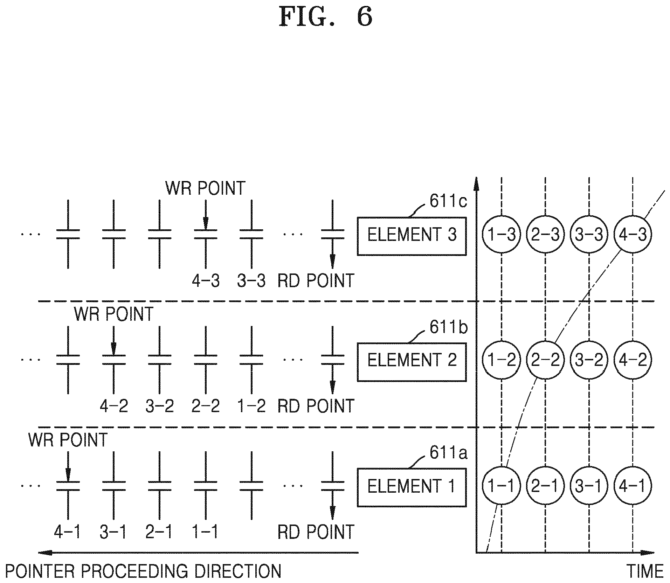

FIG. 6 is a conceptual diagram of a relationship between the number of capacitors included in the analog RAM and delay times of the ultrasound echo signals detected by each of a plurality of transducer elements, for example, first, second, and third transducer elements 611a, 611b, and 611c.

Referring to FIG. 6, each of the transducer elements 611a, 611b, and 611c may detect an ultrasound echo signal. In detail, the first transducer element 611a may first detect an ultrasound echo signal, the second transducer element 611b may then detect an ultrasound echo signal, and the third transducer element 611c may last detect an ultrasound echo signal.

A delay time needed for beamforming a detected ultrasound echo signal may correspond to a difference between a detection time of an ultrasound echo signal that is first detected and a detection time of an ultrasound echo signal that is last detected.

In the present embodiment, the first detected ultrasound echo signal is the ultrasound echo signal detected by the first transducer element 611 a, and the last detected ultrasound echo signal is the ultrasound echo signal detected by the third transducer element 611c.

In order to secure a signal delay time as long as a time corresponding to the difference between the detection time of the ultrasound echo signal detected by the first transducer element 611a and the detection time of the ultrasound echo signal detected by the third transducer element 611c, a sufficient number of capacitors need to be included in the first analog RAMs 431 corresponding to the first transducer element 611a.

The second analog RAMs 441 included in the second analog beamformer 440 need to include a sufficient number of capacitors to secure a signal delay time between the first sub-arrays 410a, 410b, and 410c corresponding to the first analog beamformers 430a, 430b, and 430c.

Referring to FIGS. 4 to 6, the number of transducer elements 411 included in each of the first sub-arrays 410a, 410b, and 410c may be determined to correspond to the number of capacitors included in the first analog RAMs 431.

For example, in order to have the time corresponding to the difference between the first detection time and the last detection time included within the delay time of the ultrasound echo signal that may be secured to correspond to the number of capacitors included in the first analog RAMs 431, the transducer elements 411 having a detection time between the transducer elements 411 corresponding to the first detection time and the last detection time may be included in each of the first sub-arrays 410a, 410b, and 410c.

Referring to FIG. 6, the first transducer element 611a detecting the ultrasound echo signal corresponding to the first detection time, the third transducer element 611c detecting the ultrasound echo signal corresponding to the last detection time, and the second transducer element 611b detecting the ultrasound echo signal having a detection time between the first detection time and the last detection time may be included in each of the first sub-arrays 410a, 410b, and 410c.

Since the transducer elements 411 having a detection time between the transducer elements corresponding to the first detection time and the last detection time are included in each of the first sub-arrays 410a, 410b, and 410c, the number of transducer elements included in each of the first sub-arrays 410a, 410b, and 410c may be determined to correspond to the number of capacitors included in the first analog RAMs 431.

Also, referring to FIGS. 4 to 6, the number of first sub-arrays 410a, 410b, and 410c may be determined to correspond to the number of capacitors included in the second analog RAMs 441. For example, the number of the first sub-arrays 410a, 410b, and 410c may be determined so that the time corresponding to the difference between the first detection time and the last detection time may be secured by the capacitors included in the second analog RAMs 441. Thus, the number of first sub-arrays may be determined to correspond to the number of capacitors included in the second analog RAMs 441.

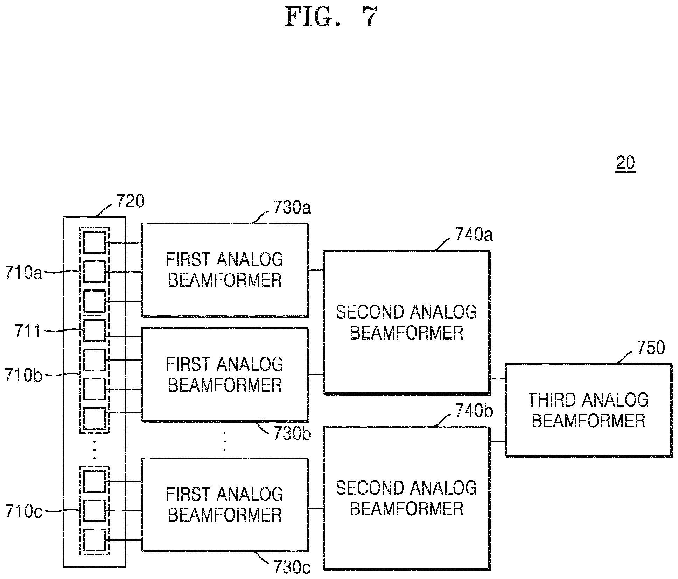

FIG. 7 is a block diagram illustrating a configuration of the probe 20 of the ultrasound diagnosis apparatus 100 including an analog beamformer of a triple structure, according to an embodiment.

The analog beamforming performed by using the analog beamformer having a dual structure according to the above-described embodiment may be extended to analog beamforming using an analog beamformer having a triple or higher structure.

Referring to FIG. 7, a plurality of transducer elements 711 may be classified to be included in a plurality of first sub-arrays 710a, 710b, and 710c. Each of a plurality of first analog beamformers 730a, 730b, and 730c may generate a first synthesized signal by performing first beamforming on an ultrasound echo signal detected by the transducer elements 711 included in each of the first sub-arrays 710a, 710b, and 710c corresponding thereto. Each of the second analog beamformers 740a and 740b may generate a second synthesized signal by performing second beamforming on the first synthesized signals generated by the first analog beamformers 730a, 730b, and 730c corresponding thereto. A third analog beamformer 750 may generate a third synthesized signal by performing third beamforming on the second synthesized signals generated by the second analog beamformers 740a and 740b corresponding thereto.

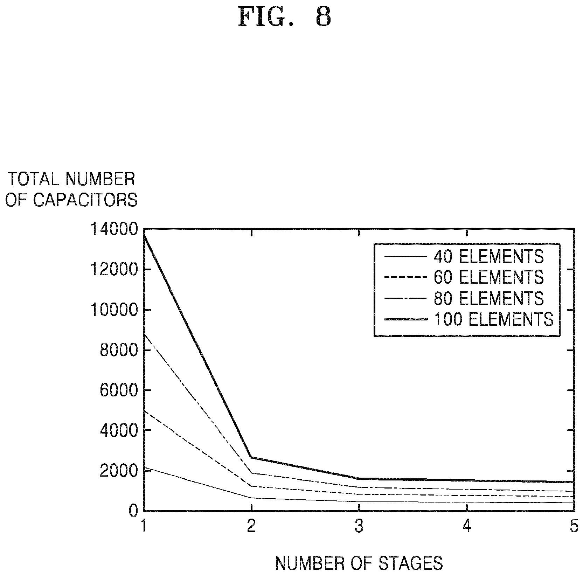

FIG. 8 is a graph of the number of capacitors needed for the analog beam forming to an analog beamformer having a multi-structure and the number of transducer elements.

Referring to FIG. 8, when beamforming is performed by using an analog beamformer having a dual structure, the number of capacitors included in the analog RAM used for analog beamforming is remarkably reduced compared to a case of performing beamforming on all transducer elements at once. When forty (40) transducer elements are needed for analog beamforming, the number of capacitors included in the analog RAM used for analog beamforming is about 2000. However, when analog beamforming is formed by using a dual structure, the number of capacitors included in the analog RAM used for analog beamforming is remarkably reduced to be less than about 500. Also, as the number of transducer elements increases, the number of capacitors included in the analog RAM used for analog beamforming when the beamforming is performed by using an analog beamformer having a multi-structure is remarkably reduced compared to a case of performing beamforming by using an analog beamformer having a single structure.

When beam forming is performed by using an analog beamformer having four or more levels (stages), the number of capacitors included in the analog RAM used for analog beamforming has almost no difference from the case of performing beamforming by using an analog beamformer having a triple structure. Thus, an analog beamformer may be configured to have a dual structure, a triple structure, or a quadruple structure by determining the number of levels of the analog beamformer effective on the reduction of the number of capacitors according to the number of transducer elements.

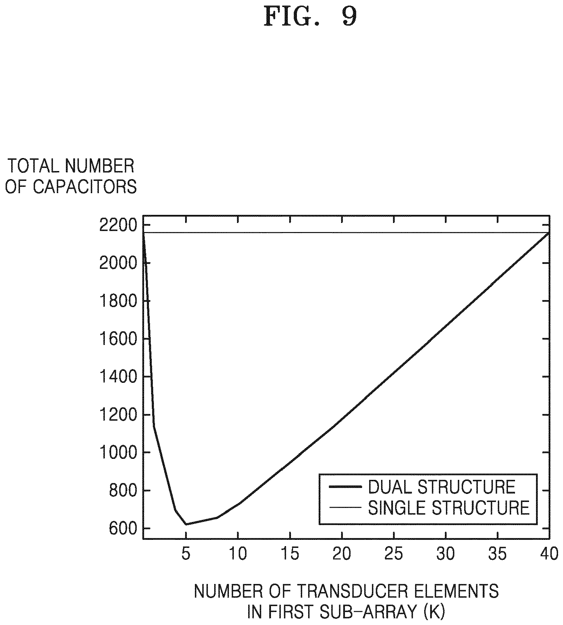

FIG. 9 is a graph of the number of capacitors needed for analog beamforming to the number of transducer elements included in a first sub-array, in a probe of an ultrasound diagnosis apparatus including an analog beamformer having a dual structure according to an embodiment.

Referring to FIG. 9, the number of capacitors included in the analog RAM used for analog beamforming when the analog beamforming is performed by using an analog beamformer having a dual structure may be reduced compared to a case of performing analog beamforming using an analog beamformer having a single structure.

In an example, when the total number of transducer elements is 40, a distance between transducer elements is 300 µm, a read-out sampling rate Fₛ of transducer elements is 10 MHz, and the number of transducer elements included in the first sub-array is 5, the number of capacitors included in the analog RAM used for analog beamforming is 624. In this case, the less number of capacitors are used, compared to a case in which the number of transducer elements in the first sub-array is designed to be different.

The number of transducer elements included in each of the first sub-arrays may be determined based on at least one of a total number of transducer elements, a distance between transducer elements, and a read-out sampling rate of transducer elements. For example, in response to an increase in the total number of transducer elements, the number of transducer elements included in each of the first sub-arrays may be increased. Also, in response to an increase in the distance between transducer elements, the number of transducer elements included in each of the first sub-arrays may be decreased. As a read-out sampling rate of transducer elements increases, the number of capacitors included in the analog RAM used for analog beamforming increases and thus the number of transducer elements included in each of the first sub-arrays may be decreased.

The number of first sub-arrays may be determined based on at least one of a total number of transducer elements, a distance between transducer elements, and a read-out sampling rate of transducer elements. For example, the number of first sub-arrays may be increased in response to an increase in the total number of transducer elements. Also, the number of first sub-arrays may be increase in response to an increase in a distance between transducer elements. Also, as a read-out sampling rate of transducer elements increases, the number of capacitors included in the analog RAM used for analog beamforming increases so that the number of first sub-arrays may be increased.

In other words, when the total number of transducer elements is 40, a distance between transducer elements is 300 µm, a read-out sampling rate Fₛ of transducer elements is 10 MHz, and the number of transducer elements included in the first sub-array is 5, the number of transducer elements included in a first sub-array and the number of first sub-arrays may be determined based on a change in a value of at least one of the total number of transducer elements, a distance between transducer elements, and a read-out sampling rate of transducer elements.

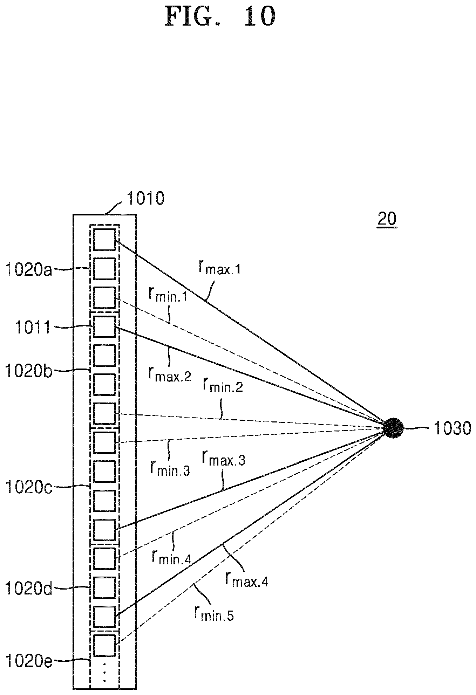

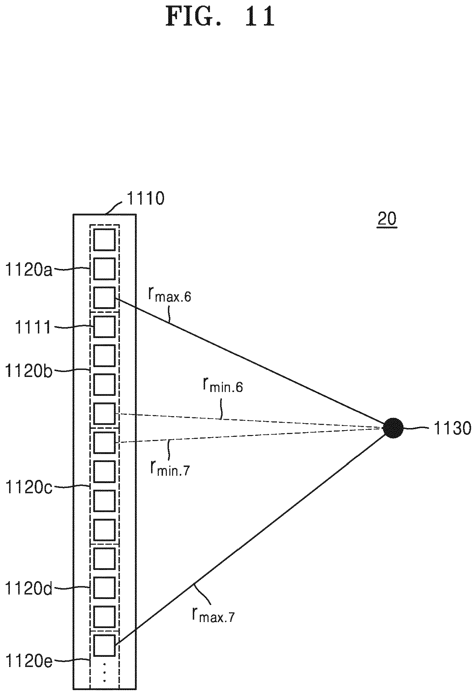

FIGS. 10 and 11 are conceptual diagrams illustrating classifications of the first sub-arrays with respect to a distance between a focal point and each of a plurality of transducer elements, in a probe of an ultrasound diagnosis apparatus including an analog beamformer having a dual structure according to embodiments.

Referring to FIGS. 10 and 11, a plurality of transducer elements may be classified to be included in a plurality of first sub-arrays based on a distance between a focal point and each of the transducer elements.

Referring to FIG. 10, a plurality of transducer elements 1011 may be classified to be included in a plurality of first sub-arrays 1020a, 1020b, 1020c, 1020d, and 1020e based on a distance from a focal point 1030.

The number of capacitors included in the analog RAM needed for accurate analog beamforming performed by an analog beamformer is proportional to a distance difference value of transducer elements having the largest distance difference from the focal point 1030 among the transducer elements 1011 on which analog beamforming is to be performed. Thus, in order to reduce the number of capacitors included in the analog RAM needed for performing accurate analog beamforming, the transducer elements 1011 may be classified to be included in the first sub-arrays 1020a, 1020b, 1020c, 1020d, and 1020e based on the distance from the focal point 1030.

In detail, a distance between the focal point 1030 and a transducer element located closest to the focal point 1030 among the transducer elements 1011 included in the first sub-array 1020a may be defined to be r*_{min.1},* and a distance between the focal point 1030 and a transducer element located farthest from the focal point 1030 among the transducer elements 1011 included in the first sub-array 1020a may be defined to be r*_{max.1}*. In this case, the number of capacitors included in the first analog RAM used for the first beamforming on the first sub-array 1020a performed by a first analog beamformer is proportional to (r*_{max.1}-r_{min.1}*)*.*

A distance between the focal point 1030 and the transducer element located closest to the focal point 1030 among the transducer elements 1011 included in the first sub-array 1020b may be defined to be r*_{min.2}*, and a distance between the focal point 1030 and the transducer element located farthest from the focal point 1030 among the transducer elements 1011 included in the first sub-array 1020b may be defined to be r*_{max.2}.* In this case, the number of capacitors included in the first analog RAM used for the first beamforming on the first sub-array 1020b performed by the first analog beamformer is proportional to (r_{*max*.*2*}-r_{*min*.*2*}).

A distance between the focal point 1030 and the transducer element located closest to the focal point 1030 among the transducer elements 1011 included in the first sub-array 1020c may be defined to be r_{*min*.*3*}, and a distance between the focal point 1030 and the transducer element located farthest from the focal point 1030 among the transducer elements 1011 included in the first sub-array 1020c may be defined to be r_{*max*.}*₃.* In this case, the number of capacitors included in the first analog RAM used for the first beamforming on the first sub-array 1020c performed by the first analog beamformer is proportional to (r*_{max.3}*-r*_{min.3}*).

A distance between the focal point 1030 and the transducer element located closest to the focal point 1030 among the transducer elements 1011 included in the first sub-array 1020d may be defined to be r_{*min*.*4*}, and a distance between the focal point 1030 and the transducer element located farthest from the focal point 1030 among the transducer elements 1011 included in the first sub-array 1020d may be defined to be r*_{max.4}*. In this case, the number of capacitors included in the first analog RAM used for the first beamforming on the first sub-array 1020d performed by the first analog beamformer is proportional to (r*_{max.4}*-r*_{min.4}*).

Thus, the number of capacitors included in the first analog RAM used for the first beamforming is determined based on the largest number among the values of (r_{*max*.*1*}-r_{*min*.*1*}), (r*_{max.2}*-r*_{min.2}*), (r*_{max.3}*-r*_{min.3}*), and (r*_{max.4}*-r*_{min.4}*).

Accordingly, the transducer elements 1011 may be included in the first sub-arrays 1020a, 1020b, 1020c, 1020d, and 1020e such that the values of (r*_{max.1}*-r*_{min.1}*), (r*_{max.2}*-x_{*min*.*2*}), (r*_{max.3}*-r_{*min*.*3*}), and (r_{*max*.*4*}-r_{*min*.*4*}) are reduced. Accordingly, the number of capacitors included in the first analog RAM used for the first beamforming may be reduced.

The transducer elements 1011 may be included in the first sub-arrays 1020a, 1020b, 1020c, 1020d, and 1020e such that a difference between the values of (r_{*max*.*1*}-r_{*min*.*1*}), (r_{*max*.*2*}-r_{*min*.*2*}), (r*_{max.3}*-r*_{min.3}*), and (r_{*max*.*4*}-r_{*min*.*4*}) are within a preset error range. Accordingly, the number of capacitors included in the first analog RAM used for the first beamforming may be reduced.

Referring to FIG. 11, a distance r_{*min*.*7*} between the focal point 1130 and the first sub-array 1120c is the shortest distance between the focal point 1130 and a plurality of first sub-arrays 1120a, 1120b, 1120c, 1120d, and 1120e. A distance r*_{max.7}* between the focal point 1130 and the first sub-array 1120e is the farthest distance between the focal point 1130 and the first sub-arrays 1120a, 1120b, 1120c, 1120d, and 1120e.

As described above, the number of capacitors included in the second analog RAM used for the second beamforming is proportional to (r*_{max.7}-*r_{*min*.*7*}).

Accordingly, the first sub-arrays 1120a, 1120b, 1120c, 1120d, and 1120e may be determined based on the distance from the focal point 1130 so that the number of capacitors included in the second analog RAM used for the second beamforming is reduced.

Also, the number of transducer elements included in the first sub-arrays may be determined based on the sum of the number of capacitors included in the first analog RAM and the number of capacitors included in the second analog RAM. For example, the number of transducer elements included in the first sub-arrays may be determined so that the sum of the number of capacitors included in the first analog RAM and the number of capacitors included in the second analog RAM is reduced. In this case, the number of transducer elements included in each of the first sub-arrays may be individually determined.

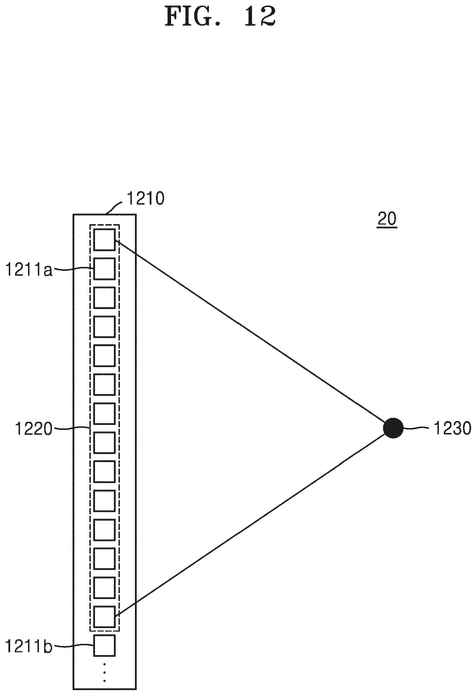

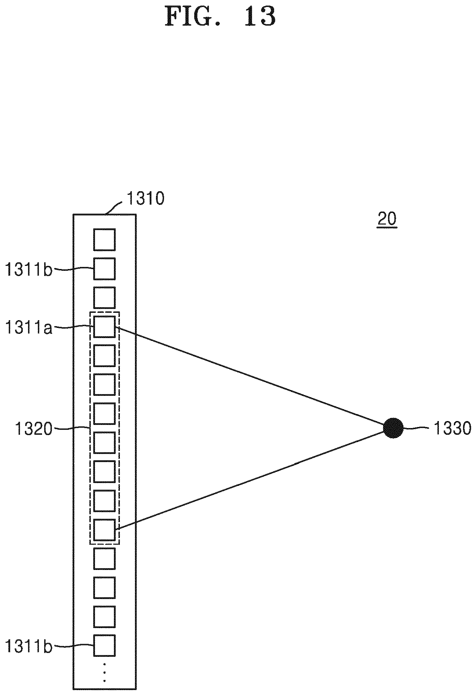

FIGS. 12 and 13 are conceptual diagrams illustrating determinations of a transducer element for detecting ultrasound echo signals based on a distance between the focal point and the transducer array, in a probe of a diagnosis apparatus according to embodiments.

Referring to FIGS. 12 and 13, transducer elements 1211a and 1311a that detect ultrasound echo signals and transducer elements 1211b and 1311b that do not detect ultrasound echo signals are determined according to a distance between focal points 1230 and 1330 and transducer arrays 1210 and 1310.

When deflection angles of the transducer elements 1211b and 1311b are preset values, for example, ±45°, and angles between the focal points 1230 and 1330 and the transducer elements 1211b and 1311b exceed a preset deflection angle, distances between the transducer elements 1211b and 1311b and the focal points 1230 and 1330 are saturated, and thus the transducer elements 1211b and 1311b are determined not to detect ultrasound echo signals. Also, even when the transducer elements 1211b and 1311b do not detect ultrasound echo signals, the ultrasound diagnosis apparatus may obtain a clear ultrasound image.

As the focal points 1230 and 1330 are located closer to the transducer arrays 1210 and 1310, the number of transducer elements 1211a and 1311a detecting ultrasound echo signals may be reduced.

In comparison between FIG. 12 and FIG. 13, for example, the focal point 1330 of FIG. 13 is located closer to the transducer array 1310 compared to the focal point 1230 of FIG. 12. The number of transducer elements 1311a detecting ultrasound echo signals of FIG. 13 may be determined to be less than the number of transducer elements 1211a detecting ultrasound echo signals of FIG. 12.

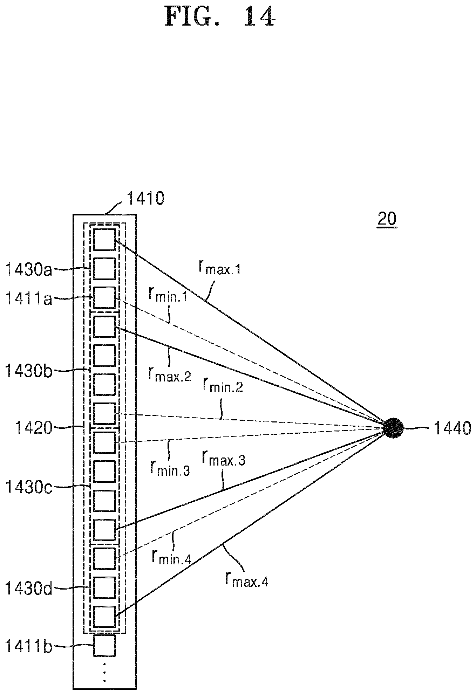

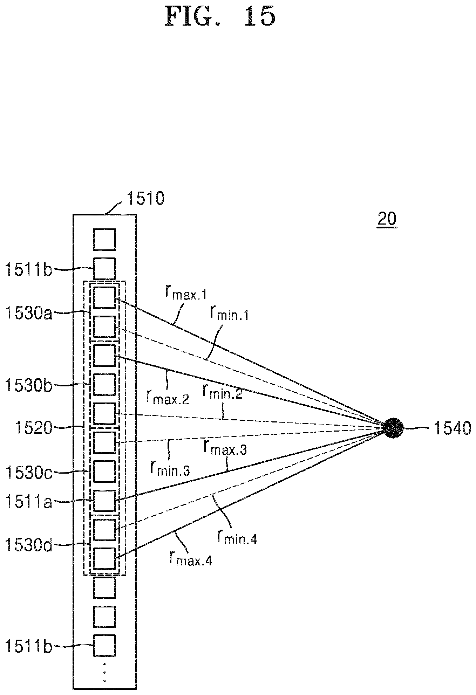

FIGS. 14 and 15 are conceptual diagrams illustrating determination of the first sub-array and the transducer element for detecting ultrasound echo signals based on a distance between the focal point and the transducer array, in a probe of a diagnosis apparatus including an analog beamformer having a dual structure according to an embodiment.

Referring to FIGS. 14 and 15, transducer elements 1411a and 1511a that detect ultrasound echo signals and transducer elements 1411b and 1511b that do not detect ultrasound echo signals may be determined based on the distances between focal points 1440 and 1540 and transducer arrays 1410 and 1510. Also, the transducer elements 1411 a and 1511a may be classified to be included in a plurality of first sub-arrays 1430a, 1430b, 1430c, and 1430d based on the distances between the focal points 1440 and 1540 and the transducer elements 1411a and 1511a detecting ultrasound echo signals.

Referring to FIGS. 14 and 15, the transducer elements 1411a and 1511a may be classified to be included in the first sub-arrays 1430a, 1430b, 1430c, and 1430d based on the distance from the focal point 1440, to reduce the number of capacitors included in the analog RAM needed for accurate analog beamforming.

In detail, a distance between the focal point 1440 and the transducer element located closest to the focal point 1440 among the transducer elements 1411a included in the first sub-array 1430a may be defined to be r*_{min.1},* and a distance between the focal point 1440 and the transducer element located farthest from the focal point 1440 among the transducer elements 1411a included in the first sub-array 1430a may be defined to be r*_{max.1}.*

In this case, the number of capacitors included in the first analog RAM used when the first analog beamformer performs first beamforming on the first sub-array 1430a is proportional to (r*ₘₐₓ-r_{min.1}*).

A distance between the focal point 1440 and the transducer element located closest to the focal point 1440 among the transducer elements 1411a included in the first sub-array 1430b may be defined to be r*_{min.2}*, and a distance between the focal point 1440 and the transducer element located farthest from the focal point 1440 among the transducer elements 1411a included in the first sub-array 1430b may be defined to be r_{*max*.}*₂.*

In this case, the number of capacitors included in the first analog RAM used when the first analog beamformer performs first beamforming on the first sub-array 1430b is proportional to (r_{*max*.*2*}-r*_{min.2}*).

A distance between the focal point 1440 and the transducer element located closest to the focal point 1440 among the transducer elements 1411a included in the first sub-array 1430c may be defined to be r*_{min.3},* and a distance between the focal point 1440 and the transducer element located farthest from the focal point 1440 among the transducer elements 1411a included in the first sub-array 1430c may be defined to be r*_{max.3}*.

In this case, the number of capacitors included in the first analog RAM used when the first analog beamformer performs first beamforming on the first sub-array 1430c is proportional to (r*_{max.3}*-r*_{min.3}*).

Also, a distance between the focal point 1440 and the transducer element located closest to the focal point 1440 among the transducer elements 1411a included in the first sub-array 1430d may be defined to be r*_{min.4}*, and a distance between the focal point 1440 and the transducer element located farthest from the focal point 1440 among the transducer elements 1411a included in the first sub-array 1430d may be defined to be r_{m*ax.4*}.

In this case, the number of capacitors included in the first analog RAM used when the first analog beamformer performs first beamforming on the first sub-array 1430d is proportional to (r*ₘₐₓ.₄*-r*_{min.4}*).

Thus, the number of capacitors included in the first analog RAM used for the first beamforming is determined in proportional to the larges value of (r*_{max.1}*-r*_{min.1}*), (r*_{max.2}*-r*_{min.2}*), (r*_{max.3}-*r*_{min.3}*), and (r_{*max*.*4*}-r*_{min.4}*).

Accordingly, the transducer elements 1411a may be classified to be included in the first sub-arrays 1430a, 1430b, 1430c, and 1430d to reduce a difference in the values of (r*_{max.1}*-r*_{min.1}*), (r*_{max.2}*-r*_{min.2}*), (r*_{max.3}-*r*_{min.3}*), and (r*_{max.4}*-r*_{min.4}*). As a result, the number of capacitors included in the first analog RAM used for the first beamforming may be reduced.

The transducer elements 1411a may be classified to be included in the first sub-arrays 1430a, 1430b, 1430c, and 1430d such that a difference in the values of (r*_{max.1}*-r*_{min.1}*), (r*_{max.2}*-r*_{min.2}*), (r*_{max.3}-*r*_{min.3}*), and (r*_{max.4}*-r*_{min.4}*) is within a preset error range. Accordingly, the number of capacitors included in the first analog RAM used for the first beamforming may be reduced.

Referring to FIG. 15, as the focal point 1540 approaches the transducer array 1510, the number of transducer elements 1511a detecting ultrasound echo signals is reduced.

The transducer elements 1511a may be included in a plurality of first sub-arrays 1530a, 1530b, 1530c, and 1530d based on the distances from the focal point 1540 such that the number of capacitors included in the analog RAM needed for accurate analog beamforming is reduced.

The transducer elements 1511a may be included in the first sub-arrays 1530a, 1530b, 1530c, and 1530d in a similar method to the method described in FIG. 14.

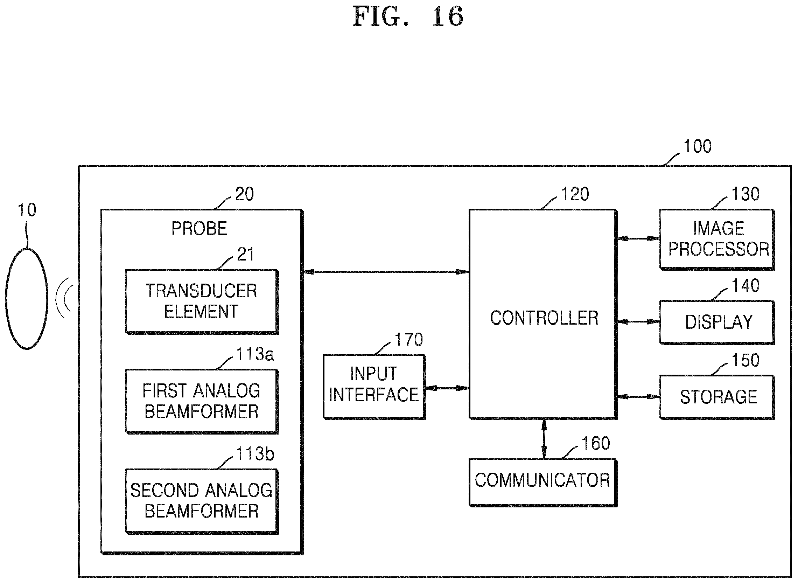

FIG. 16 is a block diagram of an ultrasound diagnosis apparatus including an analog beamformer having a dual structure according to an embodiment.

Referring to FIG. 16, the ultrasound diagnosis apparatus 100 may include the probe 20, the controller 120, the image processor 130, the display 140, the input interface 170, the storage 150, and the communicator 160.

In the present embodiment, the probe 20 may include the elements illustrated in FIGS. 3 and 4. Also, the probe 20 may include a plurality of transducer elements 21. The transducer elements 21 may be arranged in 1D or 2D, forming a transducer array. The probe 20 may include a plurality of first analog beamformers 113a and a second analog beamformer 113b.

Each of the transducer elements 21 may transmit ultrasound waves to the object 10 and detect ultrasound echo signals corresponding to the ultrasound waves reflected from the object 10 (see FIG. 1).

In the present embodiment, the controller 120 may classify the transducer elements 21 included in the probe 20 to be included in a plurality of first sub-arrays. Also, the controller 120 may match the first sub-arrays and the first analog beamformers 113a.

In the present embodiment, each of the first analog beamformers 113a may generate a first synthesized signal by performing first beamforming on each of the ultrasound echo signals detected by the transducer elements 21 included in the first sub-array.

Each of the first analog beamformers 113a may include a plurality of first analog RAMs. The first analog RAM may be used to secure a delay time between the ultrasound echo signals detected by the transducer elements 21.

The second analog beamformer 113b may generate a second synthesized signal by performing second beamforming on the first synthesized signals generated by the first analog beamformers 113a. Also, the second analog beamformer 113b may include the second analog RAMs. The second analog RAM may be used to secure a delay time between the first synthesized signals generated by the first analog beamformers 113a.

In the present embodiment, the controller 120 may determine the transducer element used to detect ultrasound echo signals based on the distance between a focal point and a transducer array including the transducer elements 21.

The controller 120 may determine the transducer elements 21 included in the first sub-array based on the distance between the focal point and the transducer elements 21.

The controller 120 may determine the number of transducer elements included in the first sub-array based on the distance between the focal point and each of the transducer elements 21.

In the present embodiment, the controller 120, the image processor 130, the display 140, the input interface 170, the storage 150, and the communicator 160 may perform the same functions as those of FIG. 1, or may further include the same elements as the controller 120, the image processor 130, the display 140, the input interface 170, the storage 150, and the communicator 160 of FIG. 1. Thus, redundant descriptions thereof are omitted.

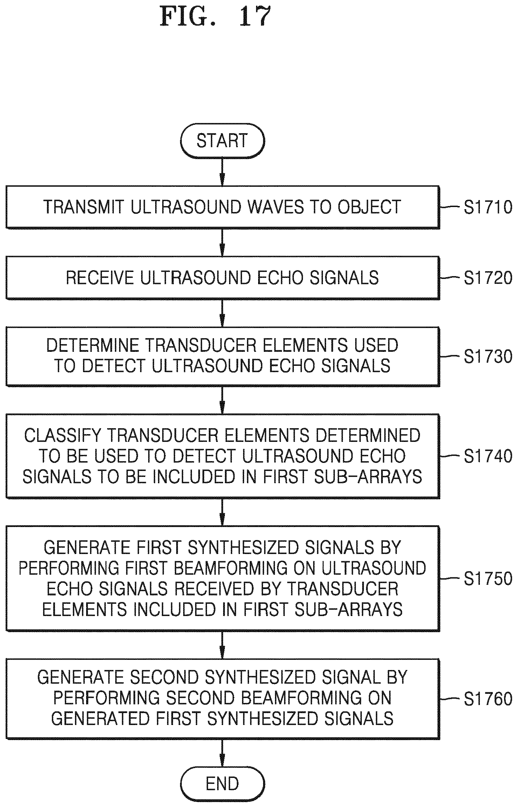

FIG. 17 is a flowchart of a method of controlling an ultrasound diagnosis apparatus including and analog beamformer having a dual structure according to an embodiment.

Referring to FIG. 17, a method of controlling an ultrasound diagnosis apparatus including an analog beamformer having a dual structure according to the present embodiment may include transmitting ultrasound waves to an object (S1710), detecting ultrasound echo signals corresponding to the ultrasound waves reflected from the object (S1720), determining transducer elements used to detect the ultrasound echo signals (S1730), classifying the transducer elements determined to be used to detect the ultrasound echo signals to be included in a plurality of first sub-arrays (S1740), generating first synthesized signals by performing first beamforming on the ultrasound echo signals detected by the transducer elements included in the first sub-arrays (S1750), and generating a second synthesized signal by performing second beamforming on the generated first synthesized signals (S1760).

In the operation S1710, the transducer elements included in the probe 20 may transmit ultrasound waves to the object by converting electronic signals to analog signals under the control of the controller 120.

In the operation S1720, the transducer elements included in the probe 20 may detect the ultrasound echo signals corresponding to the ultrasound waves transmitted to the object under the control of the controller 120.

In the operation S1730, the controller 120 may determine the transducer elements used to detect the ultrasound echo signals based on the position of the focal point on which the ultrasound waves transmitted to the object are focused. When an angle between the focal point and the transducer element exceeds a preset deflection angle, the controller 120 may determine not to use the transducer elements to detect the ultrasound echo signals. Also, as the focal point approaches the transducer array including the transducer elements, the controller 120 may determine the number of transducer elements to detect the ultrasound echo signals to be reduced.

In the operation S1740, the controller 120 may classify the transducer elements determined to detect the ultrasound echo signals to be included in the first sub-arrays. The controller 120 may determine the transducer elements included in each of the first sub-arrays and the number of first sub-arrays based on the distances between the focal point and the transducer array including the transducer elements. The controller 120 may determine the transducer elements included in each of the first sub-arrays and the number of first sub-arrays so that the number of capacitors included in the first analog RAM used for the first beamforming is reduced. The controller 120 may determine the first sub-array so that the number of capacitors included in the second analog RAM used for the second beamforming is reduced. Also, the controller 120 may determine the first sub-arrays and the transducer elements included in the first sub-arrays so that the sum of the number of capacitors included in the first analog RAM used for the first beamforming and the number of capacitors included in the second analog RAM used for the second beamforming is reduced. When the distance between the focal point and the transducer elements located farthest from the focal point among the transducer elements included in the first sub-arrays is defined to be r*ₘₐₓ*, and the distance between the focal point and the transducer elements located closest to the focal point among the transducer elements included in the first sub-arrays is defined to be r*ₘᵢₙ*, the controller 120 may determine the first sub-arrays so that the value (r*ₘₐₓ*-r*ₘᵢₙ*) of each of the first sub-arrays is reduced, and may determine the transducer elements included in the first sub-arrays.

In the operation S1750, each of a plurality of first analog beamformers may generate first synthesized signals by performing first beamforming on the ultrasound echo signals detected by the transducer elements included in each of the first sub-arrays.

In the operation S1760, the second analog beamformer may generate a second synthesized signal by receiving an input of the first synthesized signals generated by the first analog beamformer. The generated second synthesized signal is transmitted to a main body of the ultrasound diagnosis apparatus to be used for generating an ultrasound image.

The above-described embodiments of the present inventive concept may be embodied in form of a computer-readable recording medium for storing computer executable command languages and data. The command languages may be stored in form of program codes and, when executed by a processor, may perform a certain operation by generating a certain program module. Also, when executed by a processor, the command languages may perform certain operations of the disclosed embodiments.

## Claims

1. An ultrasound probe comprising:
a plurality of transducer elements configured to transmit ultrasound waves to an object and receive ultrasound echo signals corresponding to the transmitted ultrasound waves from the object, wherein the plurality of transducer elements are classified to be included in a plurality of first sub-arrays;
a plurality of first analog beamformers configured to generate first synthesized signals by performing first beamforming on each of the ultrasound echo signals received by the plurality of transducer elements included in each of the plurality of the first sub-arrays; and
a second analog beamformer configured to generate a second synthesized signal by performing second beamforming on the first synthesized signals generated by the plurality of first analog beamformers.

2. The ultrasound probe of claim 1, wherein the second analog beamformer is provided in a plural number,
the first synthesized signals generated by the plurality of first analog beamformers are classified to be input to one of the plurality of second analog beamformers,
each of the plurality of second analog beamformers generates a second synthesized signal by performing second beamforming on the first synthesized signals, and
the ultrasound probe further comprises a third analog beamformer configured to generate a third synthesized signal by performing third beamforming on the second synthesized signals generated by the plurality of second analog beamformers.

3. The ultrasound probe of claim 1, wherein each of the plurality of first analog beamformers comprises a first analog RAM including a plurality of capacitors to secure a difference in a signal delay time between the plurality of transducer elements in each of the plurality of first sub-arrays while first beamforming is performed, and
the number of the plurality of transducer elements included in each of the first sub-arrays is determined based on the number of the plurality of capacitors included in the first analog RAM.

4. The ultrasound probe of claim 1, wherein the second analog beamformer comprises a second analog RAM including a plurality of capacitors to secure a difference in a signal delay time between the plurality of first sub-arrays while second beamforming is performed, and
the number of the first sub-arrays is determined based on the number of the plurality of capacitors included in the second analog RAM.

5. The ultrasound probe of claim 1, wherein the number of the plurality of transducer elements included in each of the plurality of first sub-arrays is determined based on at least one of a total number of the plurality of transducer elements, a distance between the plurality of transducer elements, and a read-out sampling rate of the plurality of transducer elements.

6. The ultrasound probe of claim 1, wherein the number of the plurality of first sub-arrays is determined based on at least one of the number of the plurality of transducer elements, a distance between the plurality of transducer elements, and a read-out sampling rate of the plurality of transducer elements.

7. The ultrasound probe of claim 1, wherein the plurality of transducer elements are classified to be included in the plurality of first sub-arrays based on a distance between a focal point on which the ultrasound waves transmitted to the object are focused and each of the plurality of transducer elements.

8. The ultrasound probe of claim 7, wherein the plurality of first sub-arrays are determined based on a first difference value that is a difference between a maximum value and a minimum value of a distance between the focal point and each of the plurality of transducer elements included in each of the first sub-arrays.

9. The ultrasound probe of claim 8, wherein each of the plurality of first sub-arrays is determined such that the first difference value is within an error range.

10. The ultrasound probe of claim 1, wherein the plurality of first analog beamformers comprise a first analog RAM including a plurality of capacitors to secure a difference in a signal delay time between the plurality of transducer elements in each of the plurality of first sub-arrays while first beamforming is performed,
the second analog beamformer comprises a second analog RAM including a plurality of capacitors to secure a difference in a signal delay time between the plurality of first sub-arrays while second beamforming is performed, and
the number of the plurality of transducer elements included in one of the plurality of first sub-arrays is determined based on a sum of the number of the plurality of capacitors included in the first analog RAM and the number of the plurality of capacitors included in the second analog RAM.

11. The ultrasound probe of claim 1, wherein the plurality of first analog beamformers comprise a first analog RAM including a plurality of capacitors to secure a difference in a signal delay time between the plurality of transducer elements in each of the plurality of first sub-arrays while first beamforming is performed,
the second analog beamformer comprises a second analog RAM including a plurality of capacitors to secure a difference in a signal delay time between the plurality of first sub-arrays while second beamforming is performed, and
the number of the plurality of first sub-arrays is determined based on a sum of the number of the plurality of capacitors included in the first analog RAM and the number of the plurality of capacitors included in the second analog RAM.

12. A method of controlling an ultrasound diagnosis apparatus, the method comprising:
transmitting ultrasound waves to an object;
detecting ultrasound echo signals corresponding to the transmitted ultrasound waves from the object;
determining a plurality of transducer elements used to detect the ultrasound echo signals corresponding to the ultrasound waves reflected from the object based on a position of a focal point on which the ultrasound waves transmitted to the object are focused;
classifying the plurality of transducer elements determined to be included in a plurality of first sub-arrays based on the position of the focal point;
generating a plurality of first synthesized signals by performing first beamforming on each of the ultrasound echo signals detected by the plurality of transducer elements included in each of the plurality of first sub-arrays; and
generating a second synthesized signal by performing second beamforming on the first synthesized signals generated by the plurality of first analog beamformers.

13. The method of claim 12, wherein the plurality of transducer elements are classified to be included in the plurality of first sub-arrays based on a distance between the focal point on which the ultrasound waves transmitted to the object are focused and each of the plurality of transducer elements determined to be used to detect the ultrasound echo signals.

14. The method of claim 12, wherein the number of the plurality of first sub-arrays and the number of the plurality of transducer elements included in one of the plurality of first sub-arrays are determined based on the number of the plurality of transducer elements determined to be used to detect the ultrasound echo signals.

15. The method of claim 12, wherein the number of the plurality of transducer elements to detect the ultrasound echo signals decreases as the focal point approaches a transducer array comprising the plurality of transducer elements.
